# EUROPÄISCHE PATENTANMELDUNG

(11) **EP 1 025 799 A1**
(43) Veröffentlichungstag der Anmeldung: **09.08.2000**
(21) Anmeldenummer: 99126051.4
(22) Anmeldetag: 28.12.1999
(51) Int. Cl.: A61B 5/087

(54) **Atemtrainingsgerät zur Verbesserung der Vitalkapazität**

(30) Priorität: 01.02.1999 DE 19903795
(71) Anmelder: Medimex GmbH & Co. KG, 22041 Hamburg (DE)
(72) Erfinder: Boxbücher, Werner, D-44787 Bochum (DE); Zumbruch Gerhard, D-22850 Norderstedt (DE); Dubach, Werner F., CH-8124 Maur (CH)
(74) Vertreter: Siewers, Gescha, Dr.

(57) **Zusammenfassung**

Vorgesehen ist ein Atemübungsgerät, mit zwei ineinanderstehenden Kammern (10,12), wobei diese nach Maßgabe der Erfindung durch gegeneinan der verdrehfeste, oben offene zylindrische Gefäße (2,6) gebildet sind, die durch einen drehbaren Dosierdeckel (8) verschlossen sind, wobei sowohl die innere als auch die äußere Kammer mehrere Öffnungen (18,20,22,26,28) für die Einleitung, Aufteilung und Ausleitung des Inspirationsflows bzw. Exspirationsflows aufweisen, wobei durch entsprechendes Anbringen eines Atemschlauches sowie eines Stenosestopfens (54) an einer Auswahl der Öffnungen und entsprechendes Verdrehen des Dosierdeckels zur Umsteuerung der aufgeteilten Luftströme das Atemübungsgerät sowohl als Inspirations- als auch als Exspirationsübungsgerät verwendbar ist. Durch die äußere Kammer zwischen Innen- und Außenzylinder wird dabei der Hauptflow der Inspirations- bzw. Exspirationsluft geleitet.

## Beschreibung

Die vorliegende Erfindung bezieht sich auf ein Atemtrainingsgerät zur Verbesserung der Vitalkapazität, entsprechend dem Oberbegriff des unabhängigen Patentanspruches 1.

Atemtrainingsgeräte zur Verbesserung der Vitalkapazität, z.B. nach der SMI-Methode (Sustained Maximal Inspiration) sind bereits seit längerer Zeit bekannt und weit verbreitet. Nach Maßgabe dieses Verfahrens wird durch das anhaltende maximale Einatmen eine verbesserte Belüftung der Lunge eines Menschen, insbesondere der Lungenperipherie erzielt, was sich .vorteilhaft auf den Gesamtzustand des jeweiligen Menschen auswirkt.

Eine Vorrichtung zur Durchführung derartiger Einatemübungen ist beispielsweise aus dem Dokument US 4,232,683 bekannt. Dieses Dokument zeigt einen Körper mit einer Außenwand und einer Innenwand, die durch einen Durchgang voneinander separiert sind, eine Innenkammer, die durch die Innenwand definiert ist, einen Gasauslaß zur Führung eines Gases aus der Kammer, sowie eine Gaseinlaßeinrichtung, zum Einbringen von Gas in die Kammer. Eine Komponente zur Variation der Flußgeschwindigkeit des Gases durch entweder den Gaseinlaß oder den Gasauslaß und eine mit der inneren Kammer verbundene Anzeigeeinrichtung zur Anzeige der Gasflußgeschwindigkeit durch die Kammer.

Andererseits werden z. B. bei Patienten mit chronisch obstruktiven Atemwegserkrankungen Ausatemübungen gegen einen definierten Widerstand (PEP = Positive Exspiratory Pressure) empfohlen, um Sekretlockerung und Sekrettransport in den unteren Aternwegen zu fördern und die Belüftung der Lupenperipherie zu verbessern.

Der bisher bestehende Nachteil bei allen für diese beiden Atemübungen bekannten Geräten besteht darin, daß sie jeweils lediglich für eine der Übungen geeignet sind, so daß unterschiedliche Geräte angeschafft werden müssen, was sowohl eine entsprechende Logistik als auch einen entsprechenden Kostenaufwand notwendig macht.

Die Aufgabe der vorliegenden Erfindung ist es daher, eine Vorrichtung vorzusehen, die es ermöglicht, beide der vorgenannten Methoden zur Atemübung mit nur einem Gerät durchzuführen.

Diese Aufgabe wird durch die Merkmale des unabhängigen Patentanspruches 1 gelöst, wobei zweckmäßige Ausführungsformen durch die Merkmale der Unteransprüche beschrieben sind.

Vorgesehen ist ein Atemübungsgerät, mit zwei ineinanderstehenden Kammern, wobei diese nach Maßgabe der Erfindung durch gegeneinander verdrehfeste, oben offene Gefäße gebildet sind, die durch einen drehbaren Dosierdeckel verschlossen sind, wobei sowohl die innere als auch die äußere Kammer mehrere Öffnungen für die Einleitung, Aufteilung und Ausleitung des Inspirationsflows bzw. Exspirationsflows aufweisen, wobei durch entsprechendes Anbringen eines Atemschlauches sowie eines Stenosestopfens an einer Auswahl der Öffnungen und entsprechendes Verdrehen des Dosierdeckels zur Umsteuerung der aufgeteilten Luftströme das Atemübungsgerät sowohl als Inspirations- als auch als Exspirationsübungsgerät verwendbar ist. Durch die äußere Kammer zwischen Innen- und Außengefäß wird dabei der Hauptflow der Inspirations- bzw. Exspirationsluft geleitet.

Vorzugsweise sind in den zwei Kammern 6 Öffnungen vorgesehen, von denen 3 in der inneren und 3 in der äußeren Kammer ausgebildet sind, so daß jeweils 5 Öffnungen für die Einleitung, Aufteilung und Ausleitung des Inspirationsflows bzw. Exspirationsflows zur Verfügung stehen.

Ebenso bevorzugt ist das Atemübungsgerät mit einer Flowanzeige versehen, so daß der Patient auf einfache und deutliche Weise nachvollziehen kann, ob er die Übung richtig macht und den Trainingsfortschritt nachvollziehen kann. In besonders bevorzugter Weise ist die Flowanzeige durch einen in der inneren Kammer enthaltenen Indikatorball realisiert, der aufsteigt und schwebt, wenn ein bestimmter Flow herrscht.

Weiterhin bevorzugt sind die ineinander stehenden, oben offenen Gefäße als separate Bauteile vorgsehen, die derartig ausgebildet sind, daß sie nur in einer definierten Position ineinander passen. Die definierte Position der Gefäße zueinander wird vorzugsweise dadurch erreicht, daß am Innenboden des Außengefäßes und am Außenboden des Innengefäßes ineinandergreifende Stutzen vorgesehen sind, die ein kippsicheres Einsetzen des Innengefäßes in das Außengefäß gewährleisten. Die Verdrehsicherheit beider Gefäße wird dadurch erreicht, daß eine Drehsicherung aus einem Steg und einer diesen Steg aufnehmenden Aussparung vorgesehen sind. Ebenso bevorzugt weist das Innengefäß auf 180° des Umfangs einen erhöhten oberen Rand auf, der eine Öffnung in der Dichtungslippe am Dosierdeckel verschließt, wenn dieser in der Stellung Expiration steht.

Das Atemübungsgerät ist weiterhin mit einem Stenosestopfen versehen, der vorzugsweise nur in einer bestimmten Position in eine definierte Öffnung im Stutzen des Drehdeckels gesteckt werden kann und Zusatzbohrungen aufweist, die so angeordnet sind, daß sie beim Sitz des Stopfens in der einen Öffnung unwirksam sind, so daß der Stopfen eine Dichtung dieser Öffnung gewährleistet, und beim Sitz des Stopfens in der anderen Öffnung wirksam sind. Weiterhin bevorzugt sind die Querschnitte der Öffnungen des Stopfens so bemessen, daß in Zusammenwirken mit dem variablen Querschnitt der Hauptein- bzw. Auslaßöffnung ein Staudruck von ca. 15 cm H₂O bei Ausatemübungen angezeigt wird.

Es ist darüber hinaus von Vorteil, wenn ein Grobfilter zwischen Quersteg und Zapfen des Montageringes im Mundstück aspirationssicher gehalten wird.

Weitere Merkmale, Eigenschaften und Vorteile ergeben sich aus der folgenden Beschreibung eines bevorzugten Ausführungsbeispieles im Zusammenhang mit den beigefügten Zeichnungen; darin zeigt:
Fig. 1 einen schematisierten Querschnitt durch eine Ausführungsform des Atemübungsgerätes;
Fig. 2 eine Seitenansicht des äußeren Gefäßes der Ausführungsform nach Fig. 1;
Fig. 3 eine Schnittansicht des äußeren Gefäßes der Ausführungsform nach Fig. 1;
Fig. 4 eine Schnittansicht des Dosierdeckels der Ausführungsform nach Fig. 1;
Fig. 5 eine Draufsicht des Dosierdeckels der Ausführungsform nach Fig. 1;
Fig. 6 eine Seitenansicht des Dosierdeckels der Ausführungsform nach Fig. 1;
Fig. 7 eine Schnittansicht des inneren Gefäßes der Ausführungsform nach Fig. 1;
Fig. 8 eine Draufsicht des inneren Gefäßes der Ausführungsform nach Fig. 1;
Fig. 9 eine Seitenansicht des inneren Gefäßes der Ausführungsform nach Fig. 1;
Fig.. 10 eine Schnittansicht des Stenosestopfens der Ausführungsform nach Fig. 1; und
Fig. 11 eine Draufsicht des Stenosestopfens der Ausführungsform nach Fig. 1.

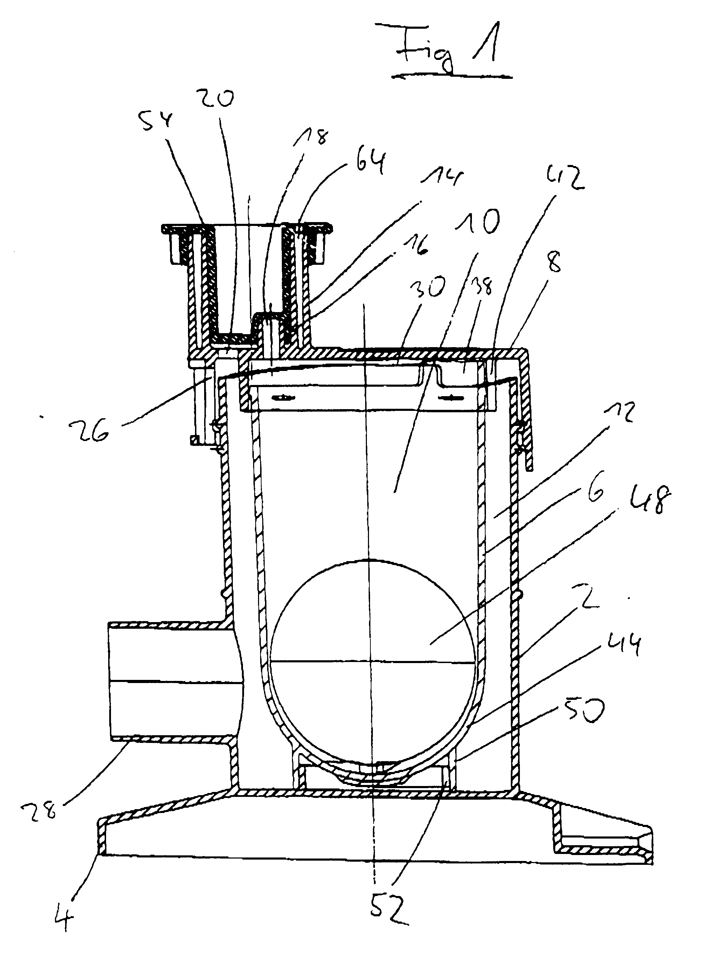

Die Fig. 1 zeigt einen schematischen Querschnitt durch eine Ausführungsform des Atemübungsgerätes. Gemäß dieser Ausführungsform besteht das Atemübungsgerät aus einem äußeren oben offenen Gefäß 2, daß an seiner Unterseite mit einem Standfuß 4 versehen ist. In diesem äußeren Gefäß 2 steht ein separat ausgebildetes und zu dem äußeren Gefäß nicht verdrehbares zweites, oben offenes Gefäß 6. Sowohl das erste Gefäß 2 als auch das in diesem verdrehfest stehende zweite Gefäß 6 sind durch einen drehbaren Dosierdeckel 8 verschlossen, so daß eine innere Kammer 10 und eine äußere Kammer 12 zwischen innerem und äußeren Gefäß entstehen.

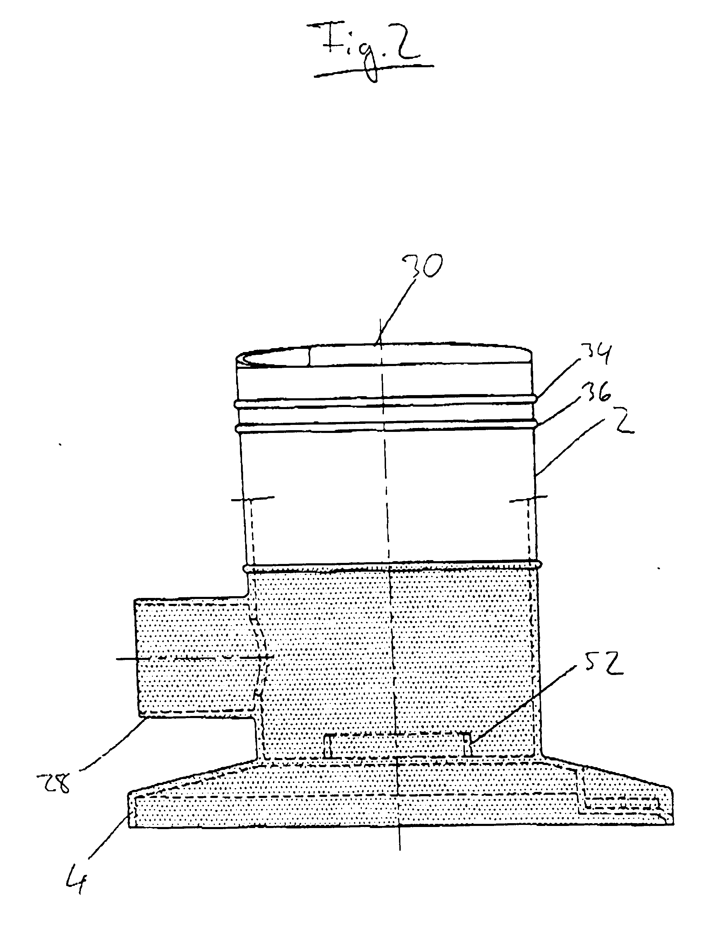

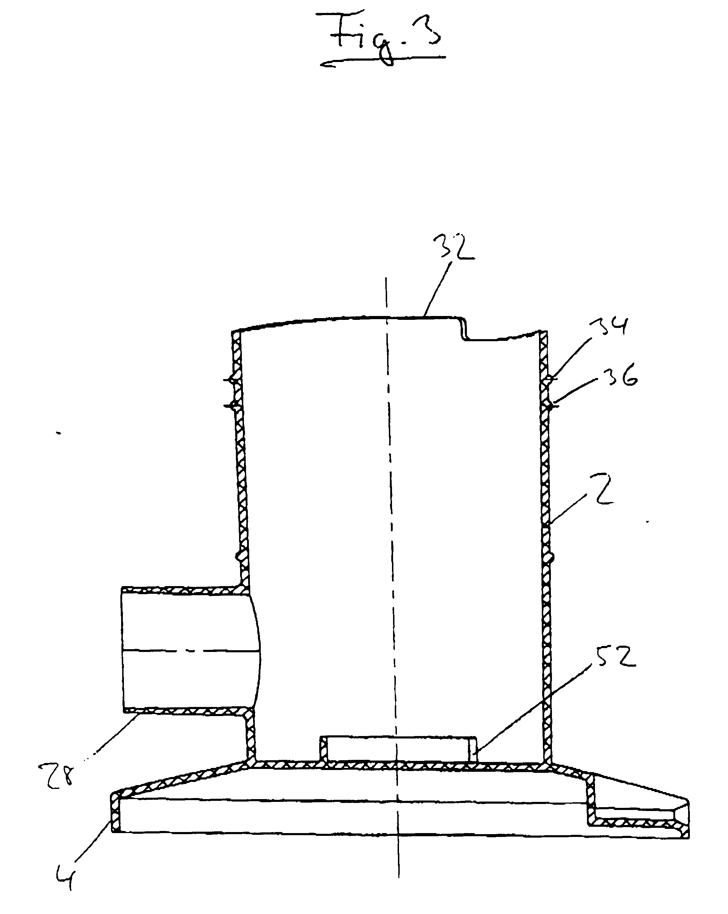

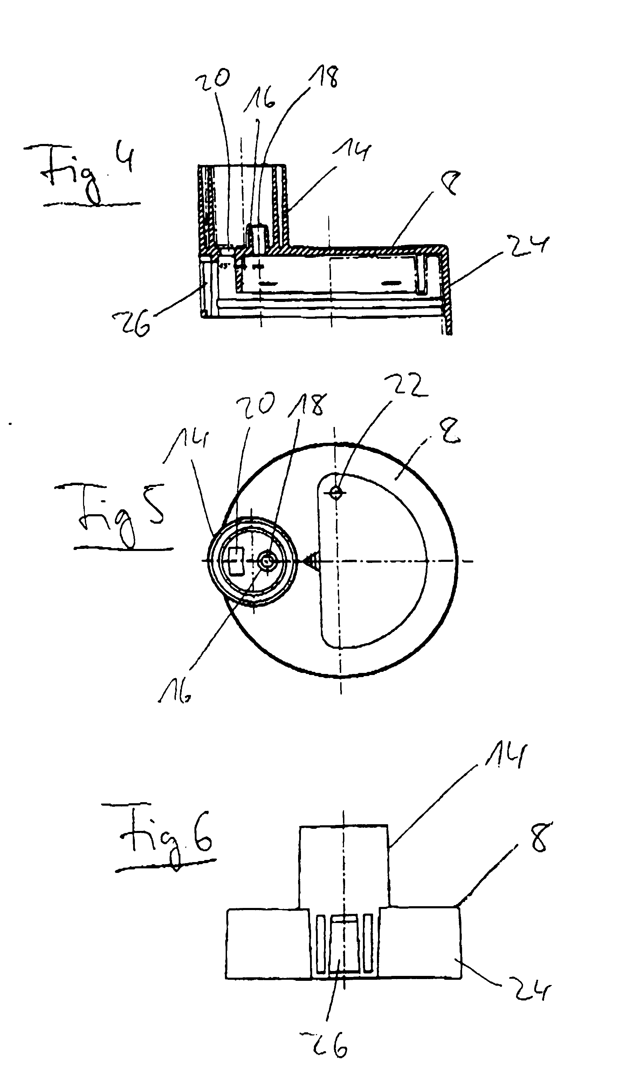

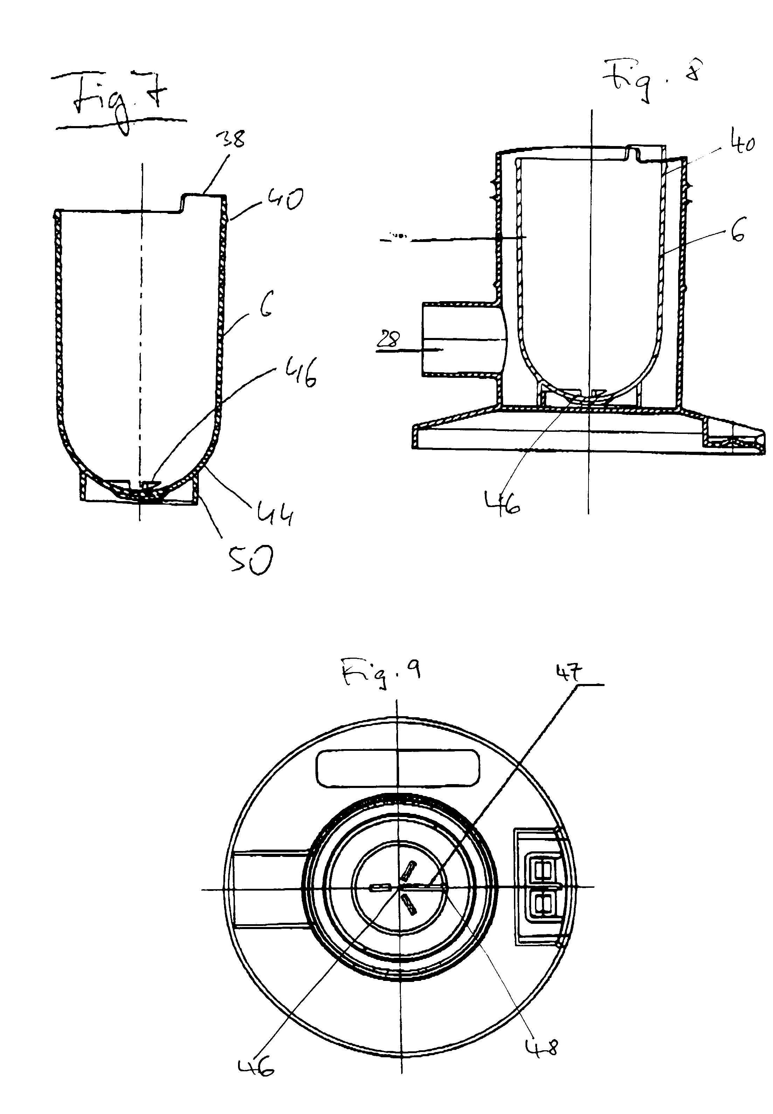

Wie der Fig. 1 im Zusammenhang mit der Fig. 4 zu entnehmen ist, ist der drehbare Dosierdeckel 8 in einem an seinen Umfang angrenzenden Bereich mit einem konischen Anschlußstutzen 14 versehen, der eine definierte Innenkontur mit einer sich vertikal erstreckenden Hülse 16 aufweist, wobei sowohl in der Hülse 16 als auch am Boden des Anschlußes 14 den Dosierdeckel 8 durchdringende Öffnungen 18 bzw. 20 vorgesehen sind. Wie der Fig. 5 zu entnehmen ist, weist der Dosierdeckel 8 zudem in dem im geschlossenen Zustand über dem inneren Gefäß liegenden Bereich eine weitere Öffnung 22 auf, durch welche das inneliegende Gefäß mit der Umgebung kommunizieren kann. Schließlich ist der Dosierdeckel 8 der vorliegenden Ausführungsform in der den oberen Randbereich des äußeren Gefäßtes 2 im geschlossenen Zustand überdeckenden Schürze 24 mit einer Öffnung 26 versehen.
Wie wiederum der Fig. 1 zu entnehmen ist, ist das äußere Gefäß 2 nach Maßgabe der vorliegenden Ausführungsform in seinem über dem Fuß 4 gelegenen Bereich mit einem weiteren Anschluß 28 versehen, dessen Innenbohrung die Wand des ersten Gefäßes 2 durchbricht. Der Fig. 1 im Zusammenhang mit den Fig. 2 und 3 ist weiterhin zu entnehmen, daß der obere Rand des ersten Gefäßes 2 in der Form zweier sich über 180° des Umfanges des Randes erstreckende Rampen 30 und 32 ausgebildet sind, die am Ende der abnehmenden Neigung vertikal ansteigen. Den Fig. 1, 2 und 3 ist weiterhin zu entnehmen, daß das äußere Gefäß 2 im Bereich der Schürze 24 des Dosierdeckels 8 mit zwei umlaufenden und vertikal voneinander beabstandeten abgerundeten Rippen 34 und 36 versehen ist, die zwischen sich eine Führung für einen entsprechenden aus der Fig. 1 ersichtlichen Vorsprung der Schürze 24 des Dosierdeckels 8 bilden, um diesen im aufgesetzten Zustand vertikal zu sichern, ohne eine Verdrehbarkeit desselben zu behindern.

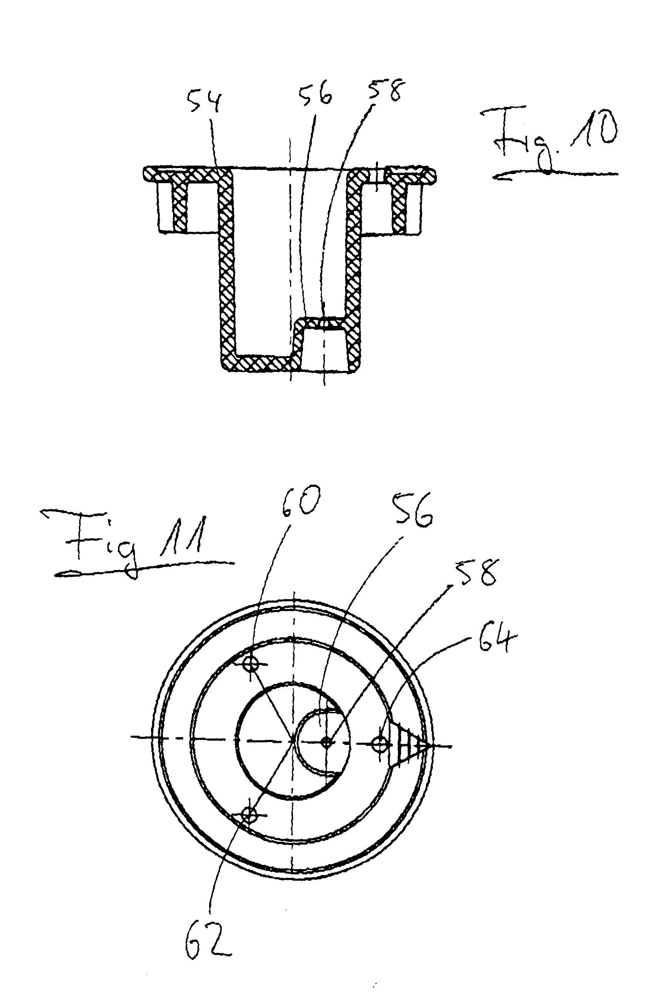

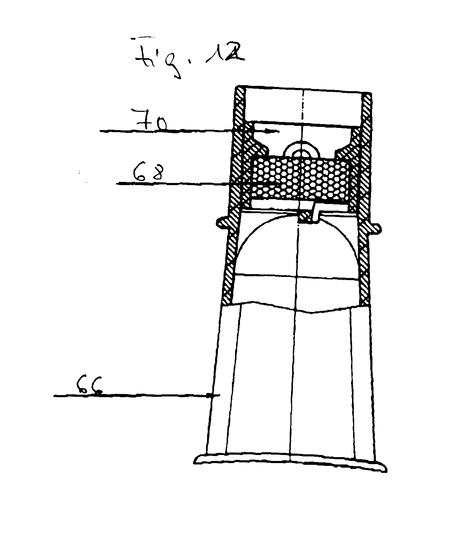

Der Fig. 1 im Zusammenhang mit den Fig. 7, 8 und 9 ist zu entnehmen, daß der obere Rand des zweiten Gefäßes 6 über 180° seines Umfanges einen vertikal vorspringenden Bereich 38 aufweist. Unmittelbar unterhalb dieses Bereiches 38 ist eine nach außen vorstehende Dichtlippe 40 vorgesehen, die in aufgesetztem Zustand des Dosierdeckels 8 in Eingriff mit einer an dessen Unterseite sich vertikal nach unten erstreckenen Dichtungslippe 42 kommt, die sich ebenfalls aus Fig. 1 im Zusammenhang mit der Fig. 4 ergibt. Aus der Fig. 1 im Zusammhang mit den Fig. 7, 8 und 9 ergibt sich darüber hinaus, daß das innere Gefäß 6 einen halbkugelförmigen Boden 44 aufweist, an dessen unterem Innenbereich drei vertikal nach oben vorstehende und eine Ebene bildende Vorsprünge 46 vorgesehen sind, die der Auflage einer in dem inneren Gefäß 6 anzuordnenden Kugel 48 dienen. An der Außenseite des halbkugelförmigen Bodens des Gefäßes 6 ist ein sich vertikal nach unten erstreckender Kreiszylinderstutzen 50 angeformt, dessen Durchmesser so gewählt ist, daß er im Paßsitz auf einem entsprechenden am Boden des äußeren Gefäßes 2 angeordneten Kreiszylinderstutzen 52 zu sitzen kommt.

Ein radial ausgerichteter Steg 47 außen am Boden des Gefäßes 6 greift in die Aussparung 48 des Kreiszylinders 52 dergestalt ein, daß nur eine Position des Gefäßes 6 zu Gefäß 2 möglich und Verdrehstabilität gewährleistet ist.

Das äußere und das innere Gefäß 2 und 6 im Zusammenhang mit dem Dosierdeckel 8 bilden daher zwei Kammern 10 und 12 mit insgesamt vier Öffnungen 18, 20, 22 und 28, wobei die Öffnung 26 in der Schürze 24 des Dosierdeckels 8 im Zusammenhang mit den Rampen 30, 32 des oberen Randes des äußeren Gefäßes 2 eine fünfte Öffnung der beiden Kammern 10 und 12, sowie der vertikal vorspringende Bereich 38 des oberen Randes des inneren Gefäßes 6 im Zusammenhang mit den Rampen 30, 32 des oberen Randes des äußeren Gefäßes 2 eine sechste Öffnung der beiden Kammern 10 und 12 bildet, wobei die innere Dichtungslippe 42 des Dosierdeckels 8 in diesem Bereich vorzugsweise eine entsprechende (nicht dargestellte) Ausnehmung aufweist.. Von den sechs Öffnungen liegen dabei drei in der inneren Kammer 10 und drei in der äußeren Kammer 12. Davon stehen abhängig von der Anordnung des noch zu beschreibenden Stenosestopfens 54 entweder in dem Anschluß 14, wie in Fig. 1 dargestellt, oder in dem Anschluß 28, jeweils fünf Öffnungen für die Einleitung, Aufteilung und Ausleitung des Inspirationsflows bzw. Exspirationsflows zur Verfügung. Die innere Kammer 10 enthält den Indikatorball 48, der aufsteigt und schwebt, wenn ein bestimmter Flow herrscht.

Der Stenosestopfen ist in den Fig. 10 und 11 beschrieben. Er ist derart aufgebaut, daß sich seine Kontur vollständig der Innenkontur des Anschlußstutzens 14 anpaßt, und dennoch auch in den Anschlußstutzen 28 einsteckbar ist. Der Stenosestopfen ist dabei mit einem die Hülse 16 umfangenden Bereich 56 versehen, der eine mit der Bohrung 18 der Hülse 16 korrespondierende Öffnung 58 aufweist. Desweiteren sind Bohrungen 60, 62, 64 vorgesehen, die beim Sitz des Stopfens 54 in dem Anschluß 14 unwirksam sind, jedoch beim Sitz des Stopfens 54 in dem Anschluß 28 wirksam sind.

Durch die äußere Kammer 12 wird der Hauptflow der Inspirations- bzw. Exspirationsluft geleitet. Die durch die Öffnung 26 in der Schürze 24 des Dosierdeckels 8 im Zusammenhang mit den Rampen 30, 32 des oberen Randes des äußeren Gefäßes 2 gebildete Öffnung der äußeren Kammer 12 kann durch Drehung des Dosierdeckels 8 in ihrem Querschnitt verändert werden. Durch Aufstecken eines (nicht dargestellten) Atemschlauches auf den Anschlußstutzen 28 und des Stenosestopfens 54 auf den Anschluß 14 sowie Drehen des Dosierdeckels 8 auf einen (nicht dargestellten) Skalenbereich "Inspiration" funktioniert der Atemtrainer als Inspirationsübungsgerät nach der SMI-Methode. Die Inspirationsluft wird durch die durch die Öffnung 26 in der Schürze 24 des Dosierdeckels 8 im Zusammenhang mit den Rampen 30, 32 des oberen Randes des äußeren Gefäßes 2 gebildete querschnittvariable Öffnung und durch die Öffnung 18 und 22 und dann durch die durch den vertikal vorspringenden Bereich 38 des oberen Randes des inneren Gefäßes 6 im Zusammenhang mit den Rampen 30, 32 des oberen Randes des äußeren Gefäßes 2 gebildete, nachgeschaltete Öffnung angesaugt. Die Öffnung 20 bleibt dabei durch den aufgesetzten Stenosestopfen 54 verschlossen. Die durch die Öffnung 26 in der Schürze 24 des Dosierdeckels 8 im Zusammenhang mit den Rampen 30, 32 des oberen Randes des äußeren Gefäßes 2 gebildete Öffnung der äußeren Kammer 12, die durch Drehung des Dosierdeckels 8 in ihrem Querschnitt verändert werden kann, reguliert die Beiluftmenge und erlaubt so eine Anpassung des Gesamtinspirationsflows auf die Leistungsfähigkeit des Patienten.

Soll das Gerät zu Exspirationstübungen verwendet werden, wird der (nicht dargestellte) Atemschlauch auf den Anschlußstutzen 14 mit den Öffnungen 18 und 20 gesteckt sowie der der Stenosestopfen 54 auf den Anschlußstutzen 28, wobei der Dosierdeckel 8 auf einen (nicht gezeigten) Skalenbereich "Exspiration" gedreht wird. Aufgrund dieser Drehung wird die durch den vertikal vorspringenden Bereich 38 des oberen Randes des inneren Gefäßes 6 im Zusammenhang mit den Rampen 30, 32 des oberen Randes des äußeren Gefäßes 2 gebildete Öffnung durch die innere Dichtungslippe 42 des Dosierdeckels 8 geschlossen. Der exspiratorische Gesamtflow teilt sich in einen Indikatorflow durch Öffnung 18 zur Öffnung 22 und den Hauptflow durch Öffnung 20 zum Anschluß 28 sowie zu der durch die Öffnung 26 in der Schürze 24 des Dosierdeckels 8 im Zusammenhang mit den Rampen 30, 32 des oberen Randes des äußeren Gefäßes 2 gebildeten und durch Drehung des Dosierdeckels 8 veränderliche Öffnung der äußeren Kammer 12.

Vorzugsweise ist der Dosierdeckel 8 so ausgelegt daß er einerseits für Inspiration oder anderseits fur Exspiration geeignet ist. Dabei sind zwei hintereinander geschaltete Skalen vorgesehen, die eine Verwechslung verhindern, d.h. es kann immer nur eine Skala (Exspiration oder Inspiration) benutzt werden. Darüber hinaus ist das gesamte Gerät so aufgebaut, daß Fehlmanipulationen weitgehend vermieden werden können. Entweder ist der Schlauch am Exspirationsstutzen, dann muss der Stenosestopfen 54 am Inspirationsstutzen sein und umgekehrt. Weiter ist nur eine Stellung des Stenosestopfens 54 möglich, da er sich sonst nicht eindrücken läßt. Auch hier wurde darauf geachtet, daß eine Fehlmanipulation, d.h. Offenstellung der falschen Löcher, vermieden wird.

Wie in Fig. 12 dargestellt, ist es vorteilhaft, wenn das Mundstück 66 einen Grobfilter 68 aufweist, der vorteilhafterweise zwischen Quersteg und Zapfen des Montageringes im Mundstück aspirationssicher gehalten wird.

## Patentansprüche

1. Atemübungsgerät, mit zwei ineinanderstehenden Kammern (10, 12), dadurch gekennzeichnet, daß diese durch gegeneinander verdrehfeste, oben offene Gefäße (2, 6) gebildet sind, die durch einen drehbaren Dosierdeckel (8) verschlossen sind, wobei sowohl die innere als auch die äußere Kammer (10, 12) mehrere Öffnungen (18, 20, 22, 26, 28) für die Einleitung, Aufteilung und Ausleitung des Inspirationsflows bzw. Exspirationsflows aufweisen, wobei durch entsprechendes Anbringen eines Atemschlauches sowie eines Stenosestopfens (54) an einer Auswahl der Öffnungen (18, 20, 28) und entsprechendes Verdrehen des Dosierdeckels (8) das Atemübungsgerät sowohl als Inspirations- als auch als Exspirationsübungsgerät verwendbar ist.

2. Atemübungsgerät nach Anspruch 1, dadurch gekennzeichnet, daß in den Kammern (10, 12) 6 Öffnungen vorgesehen sind, von denen 3 in der inneren Kammer (10) und 3 in der äußeren Kammer (12) ausgebildet sind.

3. Atemübungsgerät nach Anspruch 1 oder 2, dadurch gekennzeichnet, daß das Atemübungsgerät mit einer Flowanzeige (48) versehen ist.

4. Atemübungsgerät nach Anspruch 3, dadurch gekennzeichnet, daß die Flowanzeige durch einen in der inneren Kammer (10) enthaltenen Indikatorball (48) realisiert ist, der aufsteigt und schwebt, wenn ein bestimmter Flow herrscht.

5. Atemübungsgerät nach einem der vorhergehenden Ansprüche, dadurch gekennzeichnet, daß die ineinanderstehenden, oben offenen Gefäße (2, 6) als separate Bauteile vorgesehen sind.

6. Atemübungsgerät nach Anspruch 5, dadurch gekennzeichnet, daß am Innenboden des äußeren Gefäßes (2) und am Außenboden des inneren Gefäßes (6) ineinandergreifende konzentrische Stutzen (50, 52) vorgesehen sind, die ein kippsicheres Einsetzen der Bauteile ineinander gewährleisten.

7. Atemübungsgerät nach einem der vorhergehenden Ansprüche, dadurch gekennzeichnet, daß ein radial ausgerichteter Steg (47) außen am Boden des Gefäßes (6), der in einer Aussparung (48) des Kreiszylinders (52) eingreifen kann, vorgesehen ist, so daß nur eine Position des Gefäßes (6) zum Gefäß (2) möglich ist.

8. Atemübungsgerät nach einem der vorhergehenden Ansprüche, dadurch gekennzeichnet, daß das innere Gefäß (6) auf 180° des Umfangs einen erhöhten oberen Rand aufweist, der in eine Öffnung in einer Dichtungslippe (42) am Dosierdeckel (8) eingreift und dichtet, wenn dieser in der Stellung "Exspiration" steht.

9. Atemübungsgerät nach einem der vorhergehenden Ansprüche, dadurch gekennzeichnet, daß der Stenosestopfen (54) derart ausgebildet ist, daß er nur in einer bestimmten Position in eine definierte Öffnung (14) im Dosierdeckel (8) gesteckt werden kann, und Zusatzbohrungen (60, 62, 64) aufweist, die so angeordnet sind, daß sie beim Sitz des Stopfens (54) in der einen Öffnung (14) unwirksam sind, so daß der Stopfen eine Dichtung dieser Öffnung (14) gewährleistet, und beim Sitz des Stopfens (54) in der anderen Öffnung (28) wirksam sind.

10. Atemübungsgerät nach Anspruch 8, dadurch gekennzeichnet, daß die Querschnitte der Öffnungen (58, 60, 62, 64) des Stopfens (54) so bemessen sind, daß im Zusammenwirken mit dem variablen Querschnitt der Hauptein/auslaßöffnung ein Staudruck von ca. 15 cm H2O bei Ausatemübungen angezeigt wird.

11. Atemübungsgerät nach einem der vorhergehenden Ansprüche, dadurch gekennzeichnet, daß der Dosierdeckel (8) gegenüber dem äußeren Gefäß (2) zur Markierung der Funktionsbereiche mit einer Rasterung versehen ist.
